# EUROPEAN PATENT APPLICATION

(11) **EP 1 609 499 A1**
(43) Date of publication of application: **28.12.2005**
(21) Application number: 04710489.8
(22) Date of filing: 12.02.2004
(51) Int. Cl.: A61M 16/10

(54) **OXYGEN CONCENTRATOR FOR MEDICAL TREATMENT**

(30) Priority: 14.02.2003 JP 2003036259
(71) Applicant: Teijin Pharma Limited, Tokyo 100-0011 (JP)
(72) Inventor: NISHIMURA, Kenshi; c/o Teijin Entech Co., Ltd., Ibaraki-shi, Osaka 567-0006 (JP); MAKARI, Taizou; c/o Teijin Entech Co., Ltd., Ibaraki-shi, Osaka 567-0006 (JP); MOTOKI, Toshio; c/o Teijin Entech Co., Ltd., Ibaraki-shi, Osaka 567-0006 (JP); KOBAYASHI, H.; c/o Teijin Int. Pty. Center Ltd., Chiyoda-ku, Tokyo 100-0011 (JP)
(74) Representative: HOFFMANN EITLE
(86) International application number: PCT/JP2004/001491
(87) International publication number: WO 2004/071566

(57) **Abstract**

The present invention provides, as a pressure swing adsorption type oxygen concentrator aiming to be used for the home oxygen therapy, and having the device reliability ensuring a low failure occurrence rate, and the adsorption performance stability, ensuring the stable oxygen concentration performance even over a long period, a pressure swing type oxygen concentrator having at least one adsorption column charged with an adsorbent capable of preferentially adsorbing nitrogen rather than oxygen, and an air compressor for pressure supplying and/or vacuum exhausting air to and/or from the adsorption column, **characterized by** including a nonwoven fabric filter for collecting sealing material wear particles in an air channel between the adsorption column and the air compressor.

## Description

### TECHNICAL FIELD

The present invention relates to an oxygen concentrator for separating oxygen from air and concentrating it. More particularly, it relates to a pressure swing adsorption type oxygen concentrator particularly used for the oxygen inhalation therapy administered at home for the medical purpose.

### BACKGROUND ART

A medical oxygen concentrator for use in the home oxygen therapy is used in such a manner that air is used as a raw material, and about 21 % oxygen contained therein is concentrated to around 40 % to 90 %. For the oxygen concentration method, there are a membrane type oxygen concentrator using an oxygen enriching membrane for preferentially transmitting oxygen, and a pressure swing adsorption type oxygen concentrator using an adsorbent for preferentially adsorbing nitrogen rather than oxygen, such as zeolite, and adding air which is a raw material to an adsorption column charged with this, and adsorbing and separating nitrogen. The oxygen concentration achieved by concentration with the former oxygen concentrator is about 40 %, and about 90 % with the latter. Currently, the latter has been mainstream, and the invention relates to the latter.

Such a pressure swing adsorption type oxygen concentrator is a device of a PSA system configured as follows. A 5A type sodium zeolite, a 13X type sodium zeolite, a high performance Li zeolite which is an X type zeolite with a SiO₂/Al₂O₃ ratio of 2.0 to 3.0, and in which at least 88 % or more of AlO₄ tetrahedral units are associated with Li cations, or the like is charged into one, two, or further more adsorption columns. By means of a compressor, air is supplied thereto under pressure, to adsorb nitrogen and to separate oxygen. In addition, a channel is switched, so that the pressure is reduced to atmospheric pressure to desorb the adsorbed nitrogen. Alternatively, it is a device of a VPSA type whereby the adsorption column is reduced in pressure to vacuum state for raising the desorption regeneration efficiency. Various devices have been supplied in the market.

### DISCLOSURE OF THE INVENTION

The basic performances required of the oxygen concentrator for use in the home oxygen therapy are, other than the oxygen concentration performance, the device reliability ensuring a low failure occurrence rate, the adsorption performance stability, ensuring the stable oxygen concentration performance even over a long period, and the like, as the requirements demanded for use particularly as home medical applications. This is for the following reasons.

Namely, the oxygen concentrator for use in the home oxygen therapy is often used for 24 hours per day. Further, a patient lives depending upon the oxygen produced with this device. Therefore, the patient calls a service person instantly upon failure, and requests the repair thereof. The service person supports the requirements on a 24-hour system. It is an important requirement both for the patient and for the service person that the failure occurrence frequency of the device is low, and that the device reliability is high.

Then, in order to ensure the stable oxygen concentration performance even for a long period, an adsorption bed is required to be designed with an adsorbent in an initial charging amount of equal to or more than the necessary amount when device design is performed. This deteriorates the power consumption, and at the same time, leads to an increase in size of the device. For this reason, it is an essential requirement for ensuring the long period stability performance, to be a device showing a small variation between the initial oxygen concentration performance and the long period oxygen concentration performance.

Then, in the related art, the two requirements demanded of these oxygen concentrators are the following embodiments.

### (1) With regard to the device reliability

The major sources of failures in the device are mainly an air compressor for taking in air which is a raw material, and compressing it, and a valve portion used for pressure switching of the adsorption column of the pressure swing adsorption type oxygen concentrator.

The air compressors for use in the medical oxygen concentrator include those of a piston system, a vane system, a scroll system, and a helical system. With the device of the vane system, the vane material used is worn severely, and wear particles thereof enter the secondary side, resulting in a high failure occurrence rate of the peripheral units. In addition, the bending stress occurred in the vane material itself is also especially high. For this reason, the failure occurrence rate of the air compressor of the vane system itself is high, so that the maintenance often occurs. Therefore, those of a piston system are mainly used in the market.

As for the air compressor of the piston system, the contact area of the sealing material is also small, resulting in a small amount of wear particles of the sealing material. For this reason, the failure occurrence rate resulting therefrom is low, while it is disadvantageous in that the noise level is unfavorably high. For this noise, the noise level of the air compressor alone of the class generally used for the foregoing types of the oxygen concentrators falls within a range of 80 to 60 dBA. This is caused by the vibration of the air compressor generated in accordance with the movement of the piston and the low frequency operating sounds entailed by the torque fluctuations of an electric motor. As a means for preventing the vibration from propagating to other sites, there is known a method in which a means for achieving floating by a spring or a rubber vibration isolator as shown in JP-A-63-242901 is taken, and the sounds are enclosed in a dual sound-proof box as shown in JP-A-60-200804, JP-A-59-274654, and the like. However, it is not possible to completely enclose the sounds, and heat generated from the driving electric motor or with air compression caused in the sound-proof box is required to be cooled. Accordingly, the box is equipped with a fan. However, a noise leaks to the outside through the intake port and the exhaust port for the cooling air. For this reason, a bent passage is provided to prevent the noise leakage. Thus, various sound-proof means are taken, resulting in a large size, and large weight device. For a common medical oxygen concentrator, the weight thereof is 40 to 60 kg. It is difficult for one service person to go up and down the stairs of a building while holding the device.

The scroll system air compressor which is an air compressor achieving a lower noise is configured to have, as main components, a pair of an orbiting scroll plate and a fixed scroll plate each having an involute shape, disposed one on another in an opposing relation. Thus, by rotating the orbiting scroll, respective steps of air intake / compression / exhaust are allowed to proceed continuously. JP-A-11-92105 proposes an oxygen concentrator equipped with a scroll compressor. However, the scroll system air compressor also has a pair of chip seals disposed on the involutes. Accordingly, the seal length is large, and the amount of wear particles of the sealing material is large. As a result, wear particles enter the secondary side, resulting in a high failure occurrence rate of the peripheral units. Further, with the scroll compressor, the space formed between the involutes serves as an air leak point. This necessitates the high precision processing of the involutes. In general, three dimensional machining is required, unfavorably resulting in a very high cost. Conversely, when the processing precision is lowered for reducing the cost, the space between the involutes upon one rotation may have a large gap at one point, resulting in a large air leak, or the involutes come in contact at another point, so that the metal wear particles resulting therefrom also cause an increase in failure occurrence rate.

### (2) With regard to adsorption performance stability

The adsorption performance stability affects the deterioration state of the adsorbent performance in the adsorption column. The deterioration of the adsorbent is adversely affected by, other than moisture, deposition of foreign matters contained in air, or wear particles of the sealing material for use in the air compressor. In general, on the first side of the air compressor, an inlet filter is mounted, which prevents the foreign matters in air from entering. Conventionally, a reciprocating system air compressor causing a small amount of the sealing material to wear has been predominantly used, and hence any measures have not been taken on the secondary side of the air compressor. For this reason, with the conventional device configuration, the adsorption performance stability of the oxygen concentrator equipped with a reciprocating type air compressor is favorable. However, particularly with oxygen concentrators equipped with air compressors of a vane system, a scroll system, and a helical system classified as rotary air compressors having a feature of large seal length, it has not been possible to obtain the adsorption performance stability over a long period.

When a filter for collecting wear particles is added to the device on the secondary side of the air compressor of the oxygen concentrator, a conventional wire gauze filter immediately entails an increase in pressure due to clogging because a sufficient filtration area cannot be provided, leading to worse power consumption of the device. Therefore, it cannot be adopted. When the wire gauze is formed in pleats, or a sintered metal filter is used as the countermeasure thereagainst, an increase in size and an increase in weight of the device are unfavorably entailed. Similarly, when a general-purpose HEPA filter, or the like is adopted, the accommodation space is required to be ensured, unfavorably leading to an increase in scale of the device.

As described above, the invention provides a pressure swing adsorption type oxygen concentrator aiming to be used for the home oxygen therapy, and having a device reliability ensuring a low failure occurrence rate, and the adsorption performance stability, ensuring the stable oxygen concentration performance even over a long period. Thus, the inventors have conducted a close study on such a problem, and as a result, they found the following oxygen concentrators.

Namely, the invention provides a pressure swing type oxygen concentrator having at least one adsorption column charged with an adsorbent capable of preferentially adsorbing nitrogen rather than oxygen, and an air compressor for pressure supplying and/or vacuum exhausting air to and/or from the adsorption column, characterized by including a nonwoven fabric filter for collecting sealing material wear particles in an air channel between the adsorption column and the air compressor.

Further, the invention provides the oxygen concentrator characterized by including the nonwoven fabric filter in the air channel between a channel switching valve for switching between the adsorption and desorption steps disposed on the upstream side of the adsorption column and the air compressor, and particularly, the oxygen concentrator characterized in that an element of the nonwoven fabric filter is formed of a nonwoven fabric determined as the range of 60 to 100 % by the mass method test specified according to the ASHRAE (American Society of Heating, Refrigerating and Air-Conditioning Engineers) standard, and has a cross sectional area such that air has an average flow rate of 5 m/s or less upon passage through the same portion.

Still further, the invention provides the oxygen concentrator characterized in that the element of the nonwoven fabric filter is a nonwoven fabric of an aromatic polyamide fiber, and the invention is characterized by being applied to the oxygen concentrator in which the air compressor is a helical compressor having a helical blade, or a scroll compressor.

### BRIEF DESCRIPTION OF THE DRAWINGS

FIG. 1 shows a schematic block diagram of a dual column type oxygen concentrator of the present invention;
FIG. 2 shows a schematic block diagram of a conventional dual column type oxygen concentrator;
FIG. 3 shows a schematic cross sectional diagram of a filter for collecting wear particles of a sealing material for use in the oxygen concentrator of the invention; and
FIG. 4 shows an external view of the filter for collecting wear particles of a sealing material for use in the oxygen concentrator of the invention.

### BEST MODE FOR CARRYING OUT THE INVENTION

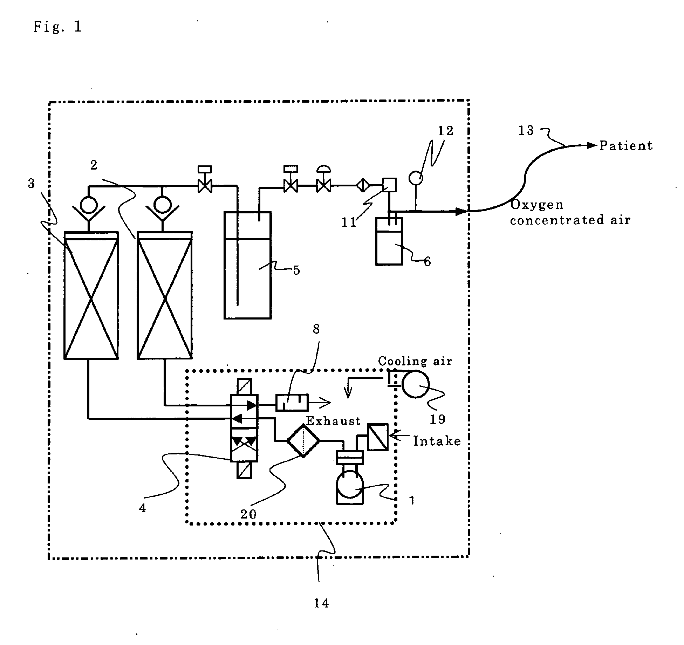

A schematic diagram of a medical oxygen concentrator of the present invention is shown in FIG. 1.

Such an oxygen concentrator is a dual column type pressure swing adsorption type oxygen concentrator configured as follows. To adsorption columns 2 and 3 charged with a 5A type sodium zeolite as an adsorbent for preferentially adsorbing nitrogen rather than oxygen, a pressurized air is successively supplied by a compressor 1, to adsorb and remove nitrogen in air. Thus, an oxygen concentrated gas with an oxygen concentration of about 90 % is extracted, and once stored in a surge tank 5. Then, oxygen is supplied therefrom via a vaporizer 6 to a patient with a respiratory organ disease who is a user.

The air compressor 1 has a compression ratio in the range of 1.5 to 3.0. The electric motor for driving the air compressor is a brushless DC electric motor having an ability of being capable of operating at 5000 rpm or less. Control of this with an inverter enables a change to a given number of revolutions.

An outside air which is a raw material air is taken in the air compressor 1 through an inlet filter 7, and pressurized air is exhausted to the adsorption column. To the adsorption column 3, pressurized air is supplied through a channel switching valve 4. Then, an adsorption step in which the adsorption column is pressurized to adsorb nitrogen in air and extracts oxygen is carried out. At the other adsorption column 2, a desorption regeneration step is carried out. Thus, the adsorption column 2 in a pressurized state is reduced in pressure to atmospheric pressure, so that the adsorbed nitrogen is released into the atmosphere through the channel switching valve 4, and an exhaust silencer 8. Such adsorption /desorption steps are carried out by successively switching the channel switching valve 4. Oxygen is thus continuously produced.

The oxygen to be supplied to a user can be supplied by switching the oxygen supply flow rate in accordance with the patient's prescribed flow rate of, for example, 1 L/min, 2 L/min, or 3 L/min by a flow rate setting unit 10.

Through a nasal cannula 13, the resulting oxygen is supplied to the patient' s nasal cavity. The oxygen is supplied to the patient in accordance with the spontaneous respiration. As a means for checking whether oxygen is supplied from the oxygen concentrator, or not, a sight flow indicator 12 such as a float is disposed.

The oxygen concentrator of the invention is characterized by including a nonwoven fabric filter 20 for collecting sealing material wear particles in the air channel between the adsorption columns and the air compressor, particularly in a duct between the exhaust side of the air compressor 1 and the channel switching valve 4. The filter 20 for collecting foreign matters is disposed on the secondary side of the air compressor 1. As a result, foreign matters such as sealing material wear particles contained in a compressed air are collected at the filter portion 20. Accordingly, purified air passes through the switching valve 4, and goes toward the adsorption columns 2 and 3.

The element 23 of the filter for collecting foreign matters is formed of a polymer type synthetic fiber nonwoven fabric of polypropylene, polyethylene, aromatic polyamide, or the like. The filtration area of the same portion is configured so that the average passing flow rate is 5 m/sec or less for the maximum passing gas amount of the same portion. When the average flow rate is 5 m/sec or more, drift of air occurs with respect to the filtration surface. Thus, such a nonwoven fabric filter cannot exhibit sufficient filtration performance.

Whereas, conceivably, the compression ratio may be high according to the operation conditions of the oxygen concentrator, so that the air temperature on the exhaust side of the air compressor is in the vicinity of 100 °C. Particularly, for the medical oxygen concentrator, the air compressor 1 is accommodated in a box 14 made of a sheet metal for the purpose of reducing the noise. Accordingly, the temperature in the box increases due to the air compressor 1 which is a heat generator. In such a case, a nonwoven fabric of an aromatic polyamide type fiber such as Nomex™ manufactured by DuPont or CONEX™ manufactured by TEIJIN Limited, having a heat resistance is preferred.

Whereas, in order to prevent the decrease in effective filtration area due to drift, the piping tubes connected through the nonwoven fabric element are desirably disposed not on a straight line, but diagonally.

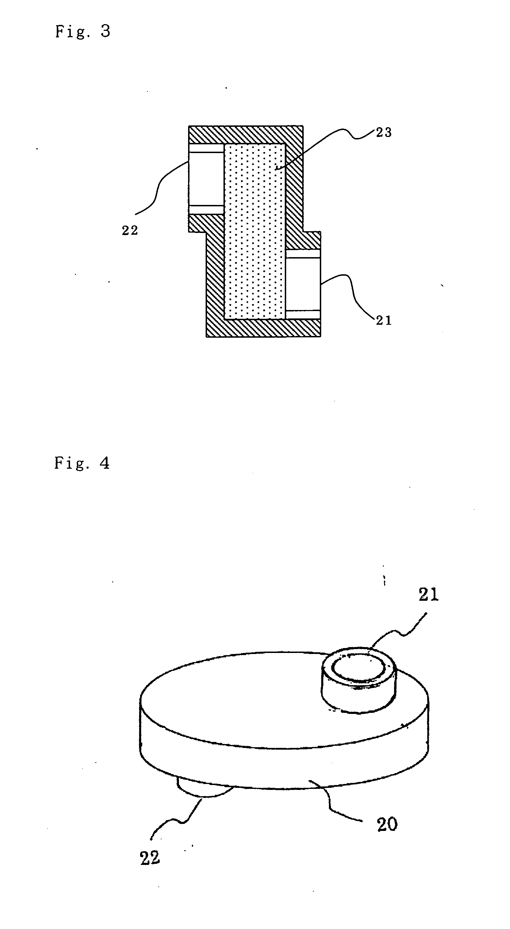

In the oxygen concentrator of the invention, as shown in FIGS. 3 and 4, a nonwoven fabric element 23 of polymetaphenylene isophthalamide (MPIA) / CONEX™ (manufactured by TEIJIN Limited) which is a meta type aromatic polyamide type heat resistant fiber is used for the cylindrical resin cartridge 20. An inlet side connection port 22 for the exhausted air from the air compressor 1 and an outlet side connection port 21 for the air after filtration are arranged diagonally.

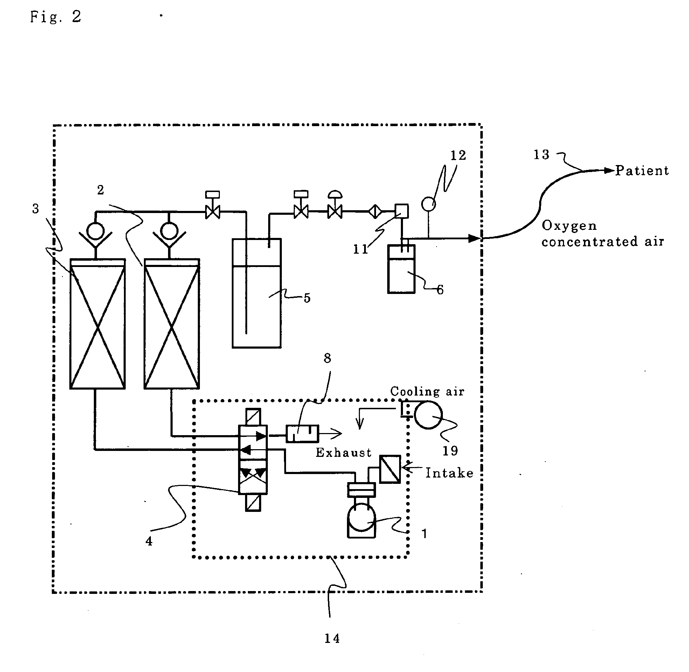

In the invention, such a nonwoven fabric filter was adopted in the oxygen concentrator as a filter for collecting wear particles of the sealing material generated from the air compressor. With an oxygen concentrator of a conventional configuration (FIG. 2), the wear particles of the sealing material generated from the air compressor 1 bite into a spool portion or the like of the switching valve 4, thereby to increase the failure occurrence rate with time. Simultaneously, the wear particles enter into the adsorption column, thereby to cause the performance deterioration of the adsorbent. In the oxygen concentrator in which the filter 20 for collecting foreign matters is mounted on the secondary side of the air compressor 1, the wear particles of the sealing material generated from the air compressor are collected by the filter. Accordingly, to the switching valve and the adsorption columns situated on the secondary side of the filter, a purified air is fed. Therefore, the failure occurrence rate is reduced, and the device reliability is increased, and at the same time, stable oxygen concentration performance can be kept even over a long period, and the adsorption performance stability can be ensured. Particularly, even for oxygen concentrators equipped with air compressors of a vane system, a scroll system, and a helical system, whereby it has been conventionally difficult to ensure the device reliability and the adsorption performance stability over a long period, such a device configuration is adopted, which can ensure the device reliability and the adsorption performance stability over a long period.

Whereas, in the invention, the element 23 of the filter for collecting the sealing material wear particles is formed of a nonwoven fabric determined as the range of 60 to 100 % by the mass method test specified according to the ASHRAE standard, and it is designed so as to have a cross sectional area such that air has an average flow rate of 5 m/sec or less upon passage through the same portion. As a result, the filter for collecting wear particles of the sealing material can be formed in compact size, and it causes a very small pressure loss of air at the same portion, and at the same time exhibits stable collecting ability over a long period.

### Examples

Below, a description will be given to Example and Comparative Example of a pressure swing adsorption type oxygen concentrator capable of supply with an oxygen concentration of 91 %, and by switching the amount of oxygen to be supplied to a flow rate of 1 L/min, 2 L/min, or 3 L/min.

### [Example 1]

In an air channel of an oxygen concentrator between two adsorption columns with a diameter of 70 mm and a length of 250 mm, charged with a 5A type sodium zeolite in a density of 0.8, and a helical type compressor (manufactured by TOSHIBA CARRIER Corporation) having a capability of providing a compressed air at a flow rate of 42 L/min when set at a maximum pressure of 120 kPa combined with a DC power-driven brushless electric motor, a filter for collecting the sealing material wear particles, formed of a nonwoven fabric element 23 made of an aromatic polyamide (manufactured by TEIJIN Limited) with an average collection rate of 90 % according to the ASHRAE mass method, and having a filtration area of 0.0025 m² in the cross-sectional area was connected as in the flow sheet of FIG. 1. The number of revolutions was set by an inverter in accordance with the flow rate 3 L/min in terms of the amount of oxygen to be supplied for continuous operation for 5000 hours.

For such a filter, the one including the air inlet connection port 22 and the exhaust connection port 21 arranged diagonally was used also as indicated by the cross sectional shape shown in FIG. 3.

### [Comparative Example 1]

Two adsorption columns with a diameter of 70 mm and a length of 250 mm, charged with a 5A type sodium zeolite in a density of 0.8, and a helical type compressor having a capability of providing a compressed air at a flow rate of 42 L/min when set at a maximum pressure of 120 kPa combined with a DC power-driven brushless electric motor were connected as shown in the flow sheet of FIG. 2. The number of revolutions was set by an inverter in accordance with the flow rate of 3 L/min in terms of the amount of oxygen to be supplied for continuous operation for 5000 hours. The other conditions used were the same conditions as in Example 1.

The results of the example and the comparative example are shown in Table 1.

The initial performances of Example 1 and Comparative Example 1 are the same. However, the difference in performances of both cases after 5000-hour continuous operation is very large. In Comparative Example 1, the oxygen concentration was reduced by 5 %, the yield was reduced by 2 %, and the power consumption increased by 5 W as compared with Example 1. Further, the number of failures is 0 for Example 1 according to the invention. In contrast, in Comparative Example 1, two failures occurred. Thus, it is indicated that, by setting the filter for collecting the sealing material wear particles on the secondary side of the air compressor, the reliability of the device is improved due to the reduction of the failure occurrence rate, and further, the adsorption performance stability over a long period is also improved.

**Table 1**

| | | Example 1 | Comparative Example 1 |
|---|---|---|---|
| Amount of oxygen supplied 3 Umin During operation (initial stage) | Air (L/min)* | 42 | 42 |
| | Concentration (%) | 91 | 91 |
| | Yield (%) | 33 | 33 |
| | Power consumption (W) | 160 | 160 |
| Amount of oxygen supplied 3 Umin During operation (after an elapse of 5000 H) | Air (L/min)* | 42 | 42 |
| | Concentration (%) | 90 | 85 |
| | Yield (%) | 32 | 30 |
| | Power consumption (W) | 160 | 165 |
| Filter | Whether used or not | Used | Not used |
| | Collection efficiency (%) | 90 | - |
| | Filtration area (cm²) | 10 | - |
| Weight (kg) | | 18 | 18 |
| Package dimensions (mm) D × W × H | | 200 × 350 × 400 | 200 × 350 × 400 |
| Adsorption columns (mm) | | diameter 70 x 250 | diameter 70 x 250 |
| 5000-H operation (number of cycles) Number of cumulative failures | | 0 | 2 |

| | | | |
|---|---|---|---|
| * The air flow rate denotes the converted flow rate at 25 °C - 1 atm. | | | |

As the oxygen concentrator of the invention, the nonwoven fabric filter 20 for collecting the sealing material wear particles is included in the air channel between the adsorption columns and the air compressor. As a result, the wear particles of the sealing material generated from the air compressor are collected by the nonwoven fabric filter. Therefore, to the switching valve and the adsorption columns situated on the secondary side of the filter, a purified air is fed. For this reason, it is possible to reduce the failure occurrence rate, and to enhance the device reliability. At the same time, it is possible to keep the stable oxygen concentration performance even over a long period, and to ensure the adsorption performance stability.

Particularly, even for oxygen concentrators equipped with air compressors of a vane system, a scroll system, and a helical system, whereby it has been conventionally difficult to ensure the device reliability and the adsorption performance stability over a long period, such a device configuration is adopted, which can ensure the device reliability and the adsorption performance stability over a long period, thus producing large effects.

## Claims

1. A pressure swing adsorption type oxygen concentrator having at least one adsorption column charged with an adsorbent capable of preferentially adsorbing nitrogen rather than oxygen, and an air compressor for pressure supplying and/or vacuum exhausting air to and/or from the adsorption column, **characterized by** including a nonwoven fabric filter for collecting sealing material wear particles in an air channel between the adsorption column and the air compressor.

2. The oxygen concentrator according to claim 1, **characterized by** including the nonwoven fabric filter in the air channel between a channel switching valve for switching between the adsorption and desorption steps disposed on the upstream side of the adsorption column and the air compressor.

3. The oxygen concentrator according to claim 1, **characterized in that** an element of the nonwoven fabric filter is formed of a nonwoven fabric determined as the range of 60 to 100 % by the mass method test according to the ASHRAE standard, and has a cross sectional area such that air has an average flow rate of 5 m/s or less upon passage through the same portion.

4. The oxygen concentrator according to claim 3, **characterized in that** the element of the nonwoven fabric filter is a nonwoven fabric of an aromatic polyamide fiber.

5. The oxygen concentrator according to claim 1, **characterized in that** the air compressor is a helical compressor having a helical blade, or a scroll compressor.
